# EUROPEAN PATENT APPLICATION

(11) **EP 1 659 175 A1**
(43) Date of publication of application: **24.05.2006**
(21) Application number: 04292734.3
(22) Date of filing: 18.11.2004
(51) Int. Cl.: C12N 15/00, A01K 67/027, A61K 48/00, C07K 14/71

(54) **Alterations in seborrheic keratoses and their applications**

(71) Applicant: Institut Curie, 75248 Paris Cedex 05 (FR); CENTRE NATIONAL DE LA RECHERCHE SCIENTIFIQUE, 75794 Paris Cedex 16 (FR)
(72) Inventor: Bernard-Pierrot, Isabelle, 94130 Nogent sur Marne (FR); Dunois-Larde, Claire, 78300 Poissy (FR); Logie, Armelle, Macclesfield, Cheshire SK11 8EQ (GB); Radvanyi, François, 92260 Fontenay aux Roses (FR); Rosty, Christophe, 75006 Paris (FR)
(74) Representative: Peaucelle, Chantal

(57) **Abstract**

The present invention relates to activated FGFR3 mutations in seborrheic keratoses and their applications for studying and treating seborrheic keratoses: use of epidermis lesion of a given transgenic animal as experimental model, treatment and composition for therapeutic and cosmetic applications.

## Description

The present invention relates to alterations in seborrheic keratoses. It also relates to their applications for studying and treating seborrheic keratoses.

Often referred to as "barnacles of life", "wisdom spots" or "age spots", seborrheic keratoses are the most common benign skin tumor in older individuals. By the age of 50, most people will develop one or more of these skin lesions. Seborrheic keratoses are not life threatening however they can be troublesome and annoying to the patient, they may become irritated and itch. Often unattractive, or even disfiguring, these skin lesions have a negative psychological impact daily reminder of aging. Although seborrheic keratosis have a variety of clinical appearances, the most typical pattern is an acanthotic layer of basal cells containing horn cysts and pseudo horn cysts. Acanthosis is accompanied by hyperkeratosis and papillomatosis. Recently, their clonal nature has been demonstrated.

However, the etiology of seborrheic keratosis has not been elucidated up to now. In particular, no molecular alteration has been yet reported in these skin lesions.

By various investigations, the inventors have surprisingly found that specific molecular alterations were associated with seborrheic keratoses. More precisely, they have found that fibroblast growth factor receptor 3 (FGFR3) mutations were involved.

FGFR3 belongs to a class of transmembrane tyrosine kinase receptors (FGFR1-4) involved in signal transduction regulating notably cell growth, differentiation, migration, wound healing and angiogenesis depending on target cell type and developmental stage.

Germinal activating mutations of FGFR3 result in craniosynostoses and dwarfing chondrodysplasias of varying severity (hypochondroplasia, achondroplasia, SADDAN and thanatophoric dysplasia). Some of these pathologies were sometimes associated with a benign skin tumor (acanthosis nigricans). Nevertheless acanthosis nigricans do not constitute the main symptom of these pathologies. Moreover, this benign skin tumor displays histological features that differ from seborrheic keratoses, in particular, no horn cysts are observed in acanthosis nigricans.

In contrast, somatic activating mutations in specific domains of FGFR3 have been shown to be associated with cancer in multiple myeloma. More recently, EP patent application 00936 765 in the names of Institut Curie and C.N.R.S., disclosed the involvement of FRGR3 in solid tumors, in particular in cancers originating from epithelial tissues (bladder and cervix carcinomas). However, other works demonstrated that up to now no mutations of FGFR3 could be identified in a series of epidermal carcinomas (squamous cell carcinomas and basal cell carcinomas).

Strikingly, the inventors have now found that somatic activating FGFR3 mutations, located in epidermis, were associated with seborrheic keratoses, and could then be used as therapeutic and/or cosmetic targets for their treatment.

"Activating mutation" refers an activating function of a mutation, which can be determined by observation of activating signals such as receptor phosphorylation or indirect effects as calcium influx, phosphorylation of target sequences.

The present invention thus relates to the use of a human or animal activating FGFR3 mutations in epidermis as therapeutic and/or cosmetic targets for seborrheic keratoses. Said targets are useful for studying or treating seborrheic keratoses.

A second object of the invention is to provide a transgenic animal with epidermis lesions as an experimental model for studying seborrheic keratoses and searching for therapeutic or cosmetic treatment thereof.

In particular, the invention concerns the use of epidermis lesions of a transgenic animal excluding human, wherein transgenic animal cells express a mutated FGFR3 gene under the control of a promoter targeting the mutated activating FGFR3 expression at the animal's epidermis.

"Epidermis lesions" designate epidermis lesions induced by the activating FGFR3 mutations.

Main histological features of the epidermis lesions observed on the transgenic animal according to the invention are acanthosis, hyperkeratosis and papillomatosis. These features are widely observed, not only in seborrheic keratoses but also in many other benign skin tumors. However, in the transgenic animal according to the invention, these features result from somatic FGFR3 mutations, which correspond to those involved in seborrheic keratoses in humans.

Activating FGFR3 mutations useful as therapeutic and/or cosmetic targets for seborrheic keratoses are in exon 7, encoding the junction between immunoglobulin-like domains II and III of FGFR3, in exon 10, encoding the transmembrane domain, in exon 15, encoding the tyrosine kinase domain I, and/or in the exon encoding the C-terminal part. Preferably, said mutations are selected in the group comprising R248C, S249C, G372C, S373C, Y375C, K652E, K652M, A393E, X809C, X809G, X809L, X809R and X809W. Codon and mutated nucleotide (nt position) are numbered according to the open reading frame of the *FGFR3b* cDNA which is the predominant form in epithelial cells.

As shown in the examples, the following mutations are targets of great interest in seborrheic keratoses: R248C, S249C, G372C, S373C, Y375C, K652E, K652M.

Surprisingly, most of the FGFR3 mutations detected in seborrheic keratoses, are identical to the germinal and somatic FGFR3 mutations described above, which always result respectively in severe genetic pathologies and in cancers, particularly in carcinomas.

As previously mentioned, the expression of the mutated FGFR3 gene in the transgenic animal is under the control of a human or animal promoter. Preferably, the promoter is selected in the group comprising Keratin 1, Keratin 5, Keratin 6, Keratin 10, Keratin 14, Involucrin, Loricrin, Transglutaminase 1 promoters.

Significant levels of transgene expression in the skin of the transgenic animal was revealed by RT-PCR analysis.

In a first embodiment, a promoter targeting the basal layer of the epidermis will be used. Preferred promoters are then Keratin 5 and Keratin 14.

Targeting the basal layer is particularly interesting since the proliferation is higher when targeting the basal cells.

In seborrheic keratoses in humans, the expression of mutated FGFR3 occurs mainly in suprabasal cells however.

Then, in a second embodiment, the transgenic will comprise a promoter targeting the suprabasal layer of the epidermis. In such an embodiment, preferred promoters are Keratin 1, Keratin 10, Involucrin and Loricrin.

Advantageously, the animal used as experimental model according to the invention is a transgenic mouse.

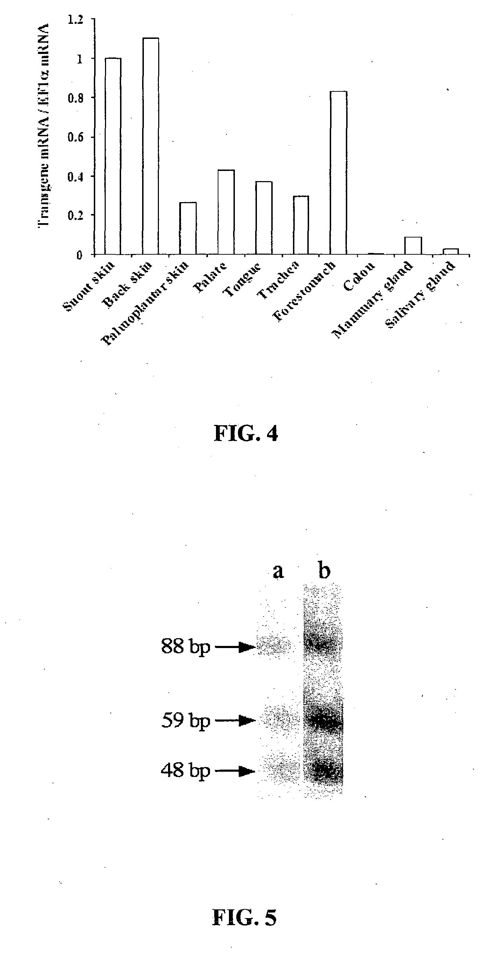

Remarkably, the human transgene and the endogenous mouse *Fgfr3* gene were expressed at similar levels in the skin of transgenic animals (see Figure 5).

Preferably, the transgenic mouse expresses FGFR3 S249C mutated gene under the control of bovine Keratin 5 or human Keratin 14 promoter. Such a model is particularly described in example 1.

The third object of the invention concerns the use of the above disclosed transgenic animal in a method for screening compounds for treating seborrheic keratoses.

The method for screening compounds for treating seborrheic keratoses comprises:
- administering a compound to be tested to the transgenic animal,
- detecting the inhibition of cell proliferation and/or the inhibition of the activation of FGFR3 and/or the resorption of the epidermis lesions,
- comparing said treated lesions with a control.

Said compound may be administered by different routes such as *per* os, intraperitoneal injection... Advantageously, a topical application will be applied on the skin lesions.

Inhibition of cell proliferation will be detected, on basal cells or supra basal cells, depending on the promoter used for expressing mutated FGFR3.

Advantageously, this method is useful for screening compounds of great interest such as tyrosine kinase inhibitors specific for FGFR3.

Another object of the invention thus relates to the use of a tyrosine kinase inhibitor specific for FGFR3 for the manufacture of a medicament for treating seborrheic keratoses.

The invention also relates to a method for treating seborrheic keratosis, comprising the step of applying to the seborrheic keratosis a composition comprising an effective amount of at least a tyrosine kinase inhibitor specific for FGFR3.

Advantageously, the tyrosine kinase inhibitor specific for FGFR3 is a chemical compound, a siRNA or an antibody. Interestingly, the use of anti-tyrosine kinase antibodies will also result in an activation of the skin complement during the immune response.

The effective amount introduced in a composition for treating seborrheic keratosis depends on the therapeutical or cosmetic use of said composition. A lower effective amount of a tyrosine kinase inhibitor specific for FGFR3 will generally be used for a cosmetic composition.

The administration route and posology of said inhibitors will be determined by one skilled in the art depending on the seborrheic keratosis to be treated (size, stage, localization...). For example, said inhibitor will be administered by applying topical treatment : ointment, balm, skin cream, gel, lotion, serum...

Other advantages and characteristics of the invention will be given in the following example wherein it will be referred to:

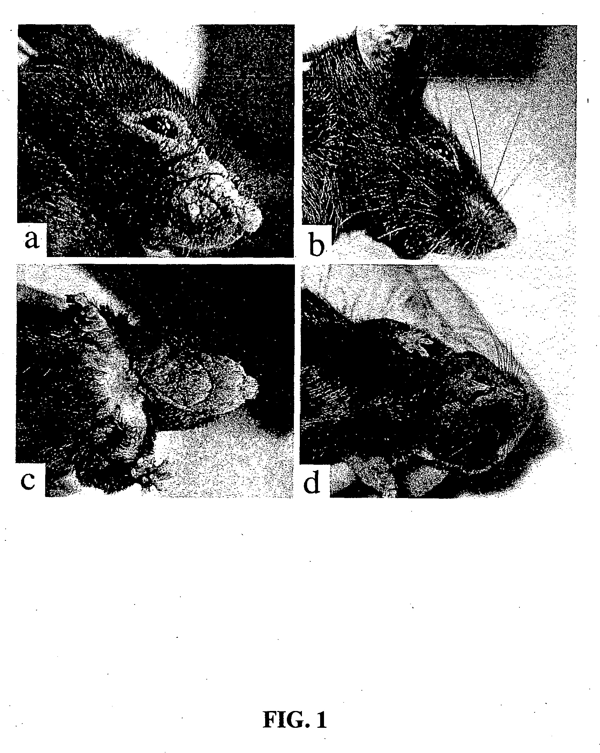

- **Figure 1:** K5-(S249C)FGFR3 transgenic mice phenotype (a, b, c, d)
   **(a)** A 6-month-old K5-(S249C)FGFR3 transgenic mouse (line #79) presenting verrucous cauliflower-like lesions on the snout and eyelids. These lesions are well demarcated, symmetric in distribution, and occasionally display partial pigmentation. (c) Throat and upper chest skin lesions on a 10-month-old K5-(S249C)FGFR3 transgenic mouse (line #79). The throat lesions resemble those on the snout in (a), areas of flat skin thickening with hair loss are clearly apparent on the upper chest. **(b, d)** Corresponding control littermates are shown on the right.

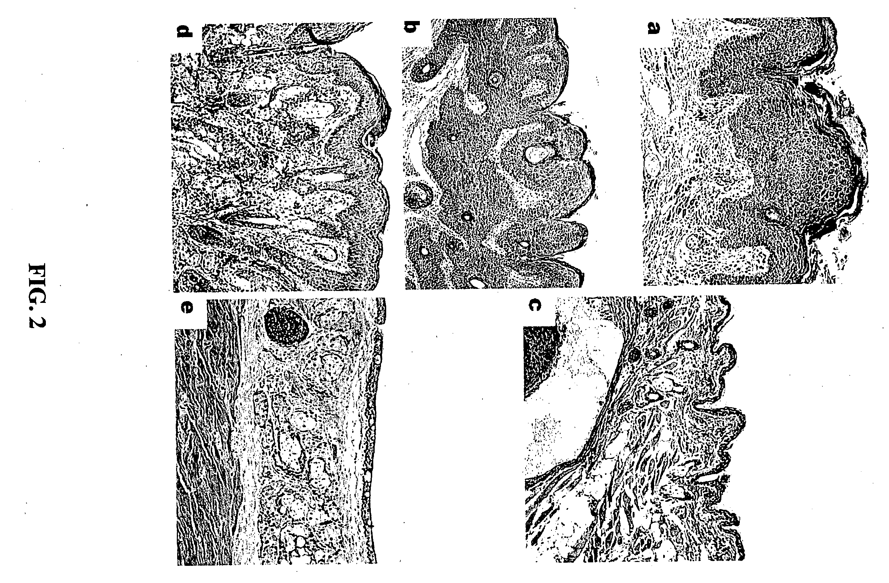

- **Figure 2:** K5-(S249C)FGFR3 transgenic mice tumor histology (a, b, c, d, e)
   **(a, b, d)** Hematoxylin, eosin and saffron sections of skin lesions from the snout (a, b), and trunk (d) of a transgenic K5-(S249C)FGFR3 mouse. **(c, e)** Sections of normal snout (c), and trunk (e), from a control littermate are shown for comparison. These skin lesions showed various degrees of acanthosis, papillomatosis and hyperkeratosis. Lesion in (b) is thicker than in (a) and shows interlacing strands (trabeculated aspect) of basaloid and squamous cells enclosing hair follicles. The parakeratosis and spongiosis observed in (a) were probably caused by itching and repetitive scratching of the lesion. Magnification x100.
- **Figure 3:** K5-(S249C)FGFR3 transgenic mice: tumor cell proliferation, as assessed by BrdU labeling.
   Mice were injected with 5-bromodeoxyuridine (BrdU) and tissue sections were analyzed by immunohistochemical staining with an antibody against BrdU. Labeled cells were restricted to the basal layer in both transgenic K5-(S249C)FGFR3 mice (a) and wild-type littermates (b). Magnification x200.
- **Figure 4:** mRNA levels for the human mutant FGFR3 S249C transgene in various tissues of K5-(S249C)FGFR3 transgenic mice.
   Expression of the human FGFR3 S249C transgene normalized with respect to a reference gene, EF1α, was quantified by real-time PCR in snout skin, back skin, palmoplantar skin, palate, tongue, trachea, forestomach, colon, mammary gland and salivary gland. In some old mice, hyperplasia of the tongue and palate epithelia was observed. No lesions were observed in other tissues.
- **Figure 5:** Relative expression levels of the human FGFR3 transgene and the endogenous mouse Fgfr3 gene in the skin of transgenic K5-(S249C)FGFR3 mice.
   Two different skin tumors cDNAs (a, b) were amplified in the presence of α[³²P]dCTP using primers recognizing both the human and mouse FGFR3 cDNAs. The 107 bp amplicons products were digested with RsaI and *Hin*P1I. The 59 bp and 48 bp bands correspond to the endogenous mouse Fgfr3; of the two bands at 88 bp and 19 bp corresponding to the human FGFR3 transgene, only the 88 bp band was visible on the gel. Quantification of the signal intensities of the various bands showed that the endogenous mouse Fgfr3 gene and the FGFR3 transgene were expressed at a similar levels.

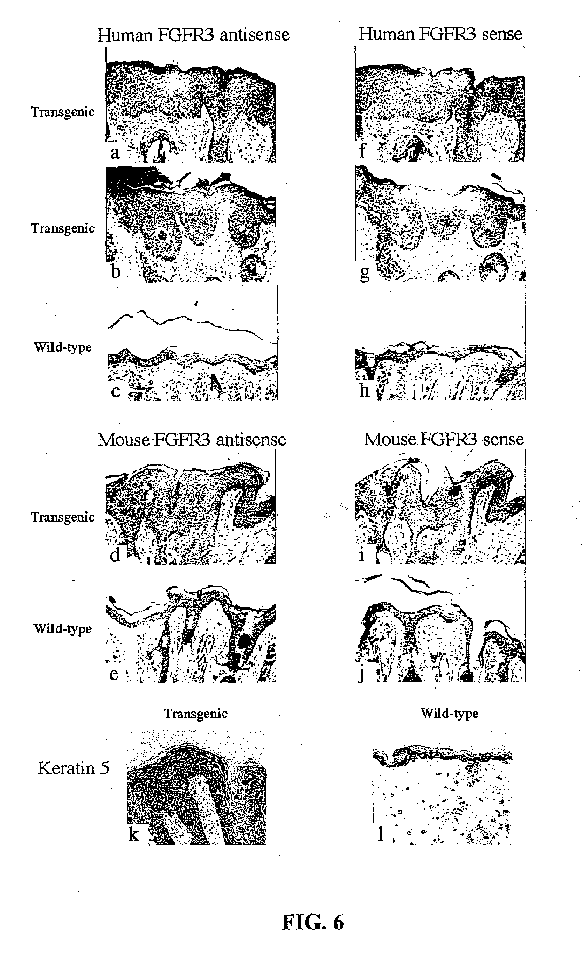

- **Figure 6:** *In situ* hybridization of human FGFR3 transgene mRNA and mouse Fgfr3 mRNA, and immunolocalization of keratin 5.
   *In situ* hybridization showed that the transgene was mainly expressed in the basal and lower suprabasal layers of the epidermis (a), with expression extending to all layers in some areas (b). As expected no transgene expression was detected in control non transgenic mouse skin (c). Endogenous mouse Fgfr3 mRNA was detected mainly in the suprabasal layers both in the transgenic (d) and control non transgenic skin (e). Hybridization to the sense riboprobes for human mutated FGFR3 (f, g, h) or mouse Fgfr3 (i, j) was used as negative control. Immunohistochemistry showed that keratin 5 had the same pattern of expression as the FGFR3 transgene mRNA: it was located mainly in the basal and lower suprabasal layers of the epidermis, with extension to all cell layers in some areas (k). In control non transgenic mouse skin, keratin 5 expression was restricted to the basal layer (1) .

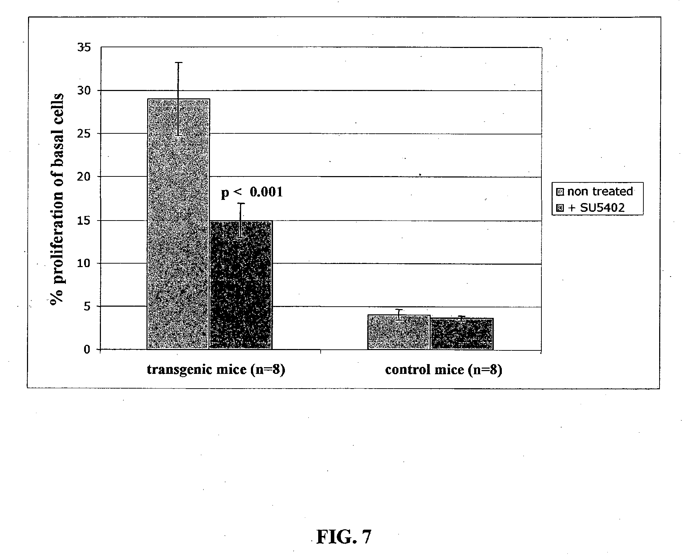

- **Figure 7:** *In vivo* effect of SU5402 on basal keratinocyte proliferation in K5.FGFR3-S249C transgenic mice.
   Snouts from 4 to 5-month-old mice who had developed skin verrucous lesion were treated on one side with 30µL acetone alone (non treated) and on the other side with 100µM SU5402 diluted in acetone (treated) three times a week for four weeks. Before sacrifice, mice were injected with 5-bromodeoxyuridine (BrdU) and tissues sections were analyzed by immunohistochemical staining with an anti-BrdU antibody. Labeled cells among 800 cells from the basal epidermis layer were numbered for each snout side.

### Example 1: Activation of the tyrosine kinase receptor FGFR3 is associated with benign skin tumors in mice and human.

### A. Materials and methods

### Generation of K5-(S249C)FGFR3 transgenic mice

The inventors targeted the expression of an activated FGFR3 carrying the S249C mutation to the epidermis in mice, using the 5' regulatory region of the bovine keratin 5 gene (Ramirez et *al. Differentiation* **58,** 53-64, 1994). The FGFR3b splice variant was used as this is the only form of FGFR3 in the murine epidermis. The S249C mutation, which causes ligand-independent activation of the receptor, is the most common mutation in carcinomas. The coding sequence of the human FGFR3b cDNA was amplified by PCR from a normal urothelium, using the primers 5'CGGGGCTGCCTGAGGAC3' (SEQ ID N°1) and 5'TGCTAGGGACCCCTCACATT3' (SEQ ID N°2). The PCR product was inserted into a cytomegalovirus promoter-driven pcDNAI/Neo expression vector (Invitrogen, San Diego, CA, USA). The S249C mutation was then introduced into this cloned FGFR3b cDNA as follows: a 729 bp region of the FGFR3b cDNA carrying the S249C mutation was amplified by RT-PCR from RNA of a bladder carcinoma, using the primers 5'CGTCGTGGAGAACAAGTTTGGCAG3' (SEQ ID N°3) and 5'CCGAGACAGCTCCCATTTG3' (SEQ ID N°4). The *Xho*1-*Aor*51H1 fragment containing the mutation was excised from the PCR product and inserted in place of the corresponding sequence in the non-mutated FGFR3b construct. The pK5-(S249C)FGFR3b construct was then obtained by inserting the mutated FGFR3b cDNA excised with *Xba*I and *Hind*III into the *Sna*BI site of the pBK5 expression vector containing the 5.2 kb bovine keratin 5 (K5) regulatory sequences, β-globin intron 2 and the 3' polyadenylation sequences of SV40. All PCR-generated segments were verified by sequencing both strands. The K5-(S249C)FGFR3 construct excised with *Sal*I and NotI was purified and microinjected into fertilized B6D2F1 oocytes. Genomic DNA was extracted from mouse tails and screened by PCR for integration of the transgene.
The care, housing, and handling of the mice conformed to the recommendations of the French Ethics Committee.

### Histology and immunohistochemistry of mouse tissues

Tissue samples from transgenic mice and wild-type littermates matched with respect to sex and body site were collected and fixed by overnight incubation at room temperature in freshly prepared Methacarn (6 vol absolute methanol / 3 vol chloroform / 1 vol acetic acid). The samples were embedded in paraffin and sections (5 µm) were cut and stained with hematoxylin-eosin-saffron for histological analysis. For immunohistochemistry, the paraffin was removed from sections, endogenous peroxidase activity was quenched and the sections were treated with 5% skim milk powder in PBS for 1 h at room temperature to block non-specific binding. The sections were then incubated overnight at 4°C with rabbit anti-keratin 5 primary antibodies (dilution 1 in 12,000) (Babco, Richmond, CA). Bound primary antibodies were detected with the EnVision™+ System (DAKO Corporation, Carpinteria, CA, USA), according to the manufacturer's instructions.

### BrdU labeling

Transgenic mice (n=5) from line #79 and control littermates (n=5) were injected (i.p.) with 0.250 mg.g⁻¹ body weight BrdU (Sigma). They were killed two hours later. BrdU immunohistochemistry was carried out with the BrdU *in situ* detection kit (BD PharMingen, San Diego, CA, USA), using the reagents and conditions suggested by the manufacturer. The number of BrdU-labeled cells among 300 cells from the basal epidermis layer was determined for each mouse when comparing the proliferation between normal and transgenic mice. 800 cells were counted when comparing the inhibitor treated and the non inhibitor treated skin lesions in the same transgenic mice.

### RT-PCR analyses

Total mouse RNA was obtained with the RNeasy mini kit (Qiagen, Courtaboeuf, France) according to the manufacturer's instructions including DNAse I treatment to avoid genomic contamination. Total RNA (1 µg) was reverse transcribed with random hexamers (20 pmol) and 200 units of MMLV reverse transcriptase. The level of expression of the human FGFR3 transgene in the various tissues of transgenic mice normalized with respect to a reference gene (EF1α) was determined by real-time PCR analysis. Primers for human FGFR3 and mouse EF1α were designed to ensure the specific amplification of a 152 bp (for FGFR3) and 219 bp (for EF1α) fragments of the cDNA. The sequences of these primers were as follows: FGFR3, forward 5' AGTCCTGGATCAGTGAGAG 3' (SEQ ID N°5), reverse 5' CTGCTCGGGGCCCGTGAACG 3' (SEQ ID N°6); EF1α, forward 5' CTGGAGCCAAGTGCTAATATGCC 3' (SEQ ID N°7), reverse 5' GCCAGGCTTGAGAACACCAGTC 3' (SEQ ID N°8). Quantitative real-time PCR was performed with the SYBR Green PCR Master Mix according to the manufacturer's instructions (Applied Biosystems). Briefly, PCR amplifications were carried out over 45 cycles, in a total volume of 25 µl. Each cDNA samples was tested in triplicate. Real-time PCR was performed on an ABI prism 7900 sequence detection system (Applied Biosystems). Following amplification, a dissociation curve was performed for all PCR products to ensure that the products generated were specific. The real-time PCR results were analyzed with the Sequence Detection System software V1.1 (Applied Biosystems). Levels of FGFR3 expression were calculated by means of the comparative Ct method with normalization to EF1α mRNA levels. This method is based on the assumption that amplification efficiency is similar for the target (human FGFR3 cDNA) and reference (EF1α cDNA) sequences. The inventors checked that this was indeed the case using dilutions of transgenic mouse skin cDNA (corresponding to 10 ng, 1 ng, 0.5 ng, 0.25 ng, 0.125 ng and 0.06 ng of reverse transcribed RNA).
They determined relative expression levels for the *FGFR3* transgene and the endogenous mouse *Fgfr3* gene in transgenic skin by amplifying the transgenic skin in the presence of α[³²P]dCTP, using the following primers: forward 5' GCAGGCATCCTCAGCTAC 3' (SEQ ID N°9) and reverse 5' TGGACTCGACCGGAGCGTA 3' (SEQ ID N°10). These primers recognized both human and mouse FGFR3 mRNA. The 107 bp amplicons obtained were digested with *Rsa*I and *Hin*P1I. The human amplicon had an *Rsa*I restriction site and the mouse amplicon a *Hin*P1I restriction site. After digestion, two fragments, 19 and 88 bp in size, were obtained from the amplified human FGFR3 cDNA and two fragments, 48 and 59 bp in size, were obtained from the amplified mouse Fgfr3 cDNA. The digested products were subjected to polyacrylamide gel electrophoresis and the intensity of the bands was determined with a Molecular Dynamics Storm PhosphorImager (Molecular Dynamics/Amersham, Sunnyvale, CA).

### In situ hybridization

The probe for the human mutated FGFR3 was derived from the 3' end of the transgene and the probe for the mouse Fgfr3 was derived from the 5' transcribed untranslated region. The probes were obtained by PCR amplification of the K5-(S249C)FGFR3 construct, using the following primers: forward 5' CACTGGTCCCCAACAATGTG 3' (SEQ ID N°11) and reverse 5' TTATGCGACTCCTGCATTAG 3' (SEQ ID N°12) (human probe) and of a mouse skin cDNA using the primers forward 5' CAATGGGCCAACAGTAGAC 3' (SEQ ID N°13) and reverse 5' TAGGCCCCAAGAAAGTCAC 3' (SEQ ID N°14) (mouse probe). The amplified products were cloned into pcRII (Invitrogen) and sequenced.
[³⁵S]UTP-labeled riboprobes were synthesized from the pcRII plasmids. The *in situ* hybridization protocol was as previously described (Sibony *et al., Lab.. Invest.* **73,** 586-591, 1995). Briefly, a microwave heating pre-treatment to enhance the hybridization signal was performed (12 minutes in a citric acid buffer in a microwave oven), followed by digestion for 20 minutes with proteinase K. Tissue sections were incubated overnight with [35S]UTP-labeled probe (approx. 10,000 cpm/µl) in a humid chamber at 50°C. Slides were washed in solutions of various degrees of stringency (from 5X SSC with 50% formamide at 55°C to 0.1X SSC at room temperature) and digested with RNase A (20 mg/ml) to remove unhybridized single-stranded RNA. Macroscopic autoradiography of the slides was obtained on Biomax film (Kodak) with an exposure time of 1-3 days, and microscopic autoradiography was carried out by dipping the slides in NTB2 liquid emulsion (Kodak). After exposure times for at least 5 weeks, slides were developed, fixed and stained with Toluidine Blue for examination under bright and dark field illumination with a Leica microscope.

### Human skin samples

Alcohol/formalin/acetic acid-fixed paraffin-embedded specimens of seborrheic keratoses were obtained from the Pathology Department of the Institut Curie and cut into 8 µm sections. One section was used for hematoxylin-eosin-saffron staining, for the identification of tumor cell areas and their subsequent microdissection from the remaining unstained sections. Genomic DNA was isolated using the QIAmp tissue kit (Qiagen), according to the manufacturer's instructions.

### Mutation analyses

Single-strand conformation polymorphism (SSCP) and sequencing was used to analyze the three regions of *FGFR3* in exons 7, 10, 15 known to harbor most of the point mutations previously described in multiple myeloma, bladder and cervix carcinomas, and skeletal dysplasias. SSCP and sequence analysis were performed as previously described (Billerey et *al., Am. J. Pathol.* **158**, 1955-1959, 2001) except that the following primer sequences were used: 5'AGTGGCGGTGGTGGTGAGGGAG3' and 5'GACGTTCCACATGTCACTGCGTGT3' for exon 7; 5'CCTCAACGCCCATGTCTTTGCAGC3' and 5'GTTCTAGAGGGCGAAGGGCGAGTTC3' for exon 10 and 5'TGGTGACCGAGGACAACGTGATG3' and 5'GTTGTGGCGGAAGGGTGTGGGA3' for exon 15.

### B. Results and discussion

The inventors investigated the role of activated FGFR3 in the epidermis by targeting the expression of an activated receptor FGFR3 S249C to the basal layer of the epidermis, using the bovine keratin 5 (K5) promoter. Three of four founders expressing the K5-(S249C)FGFR3 transgene developed a skin phenotype. These three founders were viable and fertile and transmitted the transgene to their offspring in a Mendelian fashion. The offspring developed a phenotype indistinguishable from that of the founders. At the age of three to four months, the transgenic mice presented skin verrucous tumors on the eyelids and the snout (Fig. 1a), which then gradually extended from the snout to the throat (Fig. 1c). In animals aged over five months, another type of lesion appeared on the upper trunk, characterized by a marked thickening of the skin and hair loss (Fig. 1c). None of the skin lesions arising in the various transgenic lines showed any signs of regression.

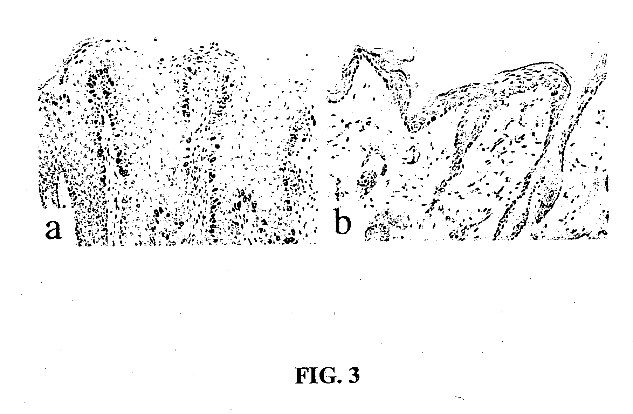

Histological examination of the skin lesions revealed common features, irrespective of body site and gross appearance: i) pronounced acanthosis (thickening of the nucleated layers of the epithelium), ii) hyperkeratosis (thickening of the stratum corneum) with occasional parakeratosis (hyperkeratosis, with retention of nuclei), iii) papillomatosis (numerous projections of the dermal papillae) (Fig. 2a, 2b, 2d). The most exophytic lesions of the snout displayed a trabeculated pattern, with rare keratin-filled invaginations and small keratin cysts, which generally surrounded a hair shaft (Fig. 2b). No abnormalities of the hair follicles or sebaceous glands were observed. No histological features of malignancy were detected: mitotic figures were rare and always localized in the basal layer of the epidermis, cytological atypia was not observed. The absence of malignancy was evident, even in the lesions of transgenic mice more than 18 months old. Cell proliferation was studied by incorporation of the thymidine analog 5-bromo-2-deoxyuridine (BrdU). Consistent with the benign nature of the lesions, proliferating cells were restricted to the basal cell layer of the epidermis and to hair follicles in both transgenic and control mice (Fig.3). However, the proportion of basal cells undergoing proliferation was significantly higher in transgenic mouse epidermis than in control skin (27% versus 1.8%, p < 0.0001).

RT-PCR analysis revealed significant levels of transgene expression in the skin of K5-(S249C)FGFR3 transgenic mice. FGFR3 transgene expression was also detected in the palate, tongue, trachea, forestomach, mammary gland and salivary gland of transgenic animals consistent with the keratin 5 promoter driving transgene expression in several epithelial tissues (Fig.4). The human transgene and the endogenous mouse Fgfr3 gene were expressed at similar levels in the skin of transgenic animals (Fig.5). *In situ* hybridization showed that the transgene was mainly expressed in the basal and lower suprabasal layers of the epidermis, with expression extending to all layers in some areas (Fig.6). This pattern corresponded to the distribution of keratin 5 expression in transgenic skin, as demonstrated by immunohistochemistry (Fig.6). By *in* situ hybridization endogenous mouse Fgfr3 mRNA was detected with the strongest signal in the suprabasal layers in both transgenic and control skin (Fig.6).

The skin lesions observed in transgenic mice expressing mutated *FGFR3* displayed several histological features (acanthosis, hyperkeratosis, papillomatosis) common to benign human epidermal tumors including seborrheic keratosis, one of the most common benign epidermal tumors in humans. The inventors carried out SSCP analysis, followed by DNA sequencing of the *FGFR3* gene in 62 seborrheic keratosis samples. They identified missense mutations in 24 samples (39%) (see Table 1 and Table 2).

**Table 1 FGFR3 mutations in seborrheic keratoses**

| Codon | nt position | Exon | Mutation | Predicted effect | Number of tumors |
|---|---|---|---|---|---|
| 248 | 742 | 7 | CGC → TGC | Arg → Cys | 5 |
| 249 | 746 | 7 | TCC → TGC | Ser → Cys | 5 |
| 372 (370) | 1114 | 10 | GGC → TGC | Gly → Cys | 3 |
| 373 (371) | 1117 | 10 | AGT → TGT | Ser → Cys | 3 |
| 375 (373) | 1124 | 10 | TAT → TGT | Tyr → Cys | 4 |
| 652 (650) | 1954 | 15 | AAG → GAG | Lys → Glu | 1 |
| 652 (650) | 1955 | 15 | AAG → ATG | Lys → Met | 3 |

Codon and mutated nucleotide (nt position) are numbered according to the open reading frame of the *FGFR3b* cDNA. This isoform, which is the predominant form in epithelial cells, contains two more amino acids than the FGFR3c isoform, which is found in bone. The codon numbers according to the open reading frame of the FGFR3c cDNA are indicated in brackets.

**Table 2 Clinical data and FGFR3 mutations**

| **Case #** | Age | **Sex** | **Localization** of the tumor | **Size** (mm) | **Histological** Type | *FGFR3* Mutation |
|---|---|---|---|---|---|---|
| 1 | 64 | F | Face | 10 | Acanthotic | R248C |
| 2 | 51 | M | Face | 8 | Acanthotic | S373C |
| 3 | 54 | M | Face | 9 | Hyperkeratotic | R248C |
| 4 | 76 | F | Trunk | 9 | Acanthotic | S249C |
| 5 | 91 | F | Trunk | 12 | Acanthotic | S249C |
| 6 | 91 | F | Trunk | 15 | Acanthotic | Y375C |
| 7 | 54 | F | Face | 10 | Acanthotic | G372C |
| 8 | 84 | F | Trunk | 8 | Hyperkeratotic | R248C |
| 9 | 43 | F | Face | 12 | Acanthotic | K652M |
| 10 | 67 | F | Face | 6 | Acanthotic | G372C |
| 11 | 72 | F | Trunk | 13 | Acanthotic | Y375C |
| 12 | 85 | M | Trunk | 6 | Acanthotic | R248C |
| 13 | 67 | F | Face | 8 | Acanthotic | S249C |
| 14 | 31 | F | Trunk | 3 | Acanthotic | R248C |
| 15 | 83 | F | Trunk | 6 | Hyperkeratotic | G372C |
| 16 | 31 | F | Trunk | 5 | Acanthotic | K652M |
| 17 | 79 | M | Face | 11 | Hyperkeratotic | S373C |
| 18 | 67 | F | Trunk | 6 | Acanthotic | Y375C |
| 19 | 65 | F | Trunk | 15 | Hyperkeratotic | S373C |
| 20 | 79 | F | Trunk | 7 | Hyperkeratotic | K652M |
| 21 | 70 | F | Face | 15 | Acanthotic | S249C |
| 22 | 79 | F | Face | 6 | Hyperkeratotic | K652E |
| 23 | 31 | M | Face | 4 | Acanthotic | Y375C |
| 24 | 75 | M | Face | 10 | Hyperkeratotic | S249C |

F: female; M: male; wt: wild-type

Remarkably, all of the mutations identified (R248C, S249C, G372C, S373C, Y375C, K652E, K652M) were identical to the somatic mutations previously identified in bladder and cervix carcinomas and to the germinal mutations responsible for thanatophoric dysplasia, a lethal form of dwarfism, and SADDAN syndrome, a severe form of dwarfism. Direct sequencing of matched constitutional DNA, available for 10 individuals with seborrheic keratosis displaying *FGFR3* mutations, revealed wild-type sequences, demonstrating the somatic nature of these mutations.

The skin lesions observed in the transgenic mouse model have several histological features in common with seborrheic keratoses (acanthosis, hyperkeratosis, papillomatosis). Keratin-filled cysts were present in some thick lesions but differed from the classical horn cysts or pseudo-horn cysts of human seborrheic keratosis in that they surrounded a hair. The observed differences might be due to a different pattern of expression of the transgene and the endogenous FGFR3: the expression of the transgene was predominant in the basal layer and the first suprabasal layers whereas the endogenous FGFR3 was more expressed in the suprabasal layers both in murine and human epidermis. Thus, expressing mutated FGFR3 in the suprabasal compartment will provide a model closer to human seborrheic keratosis than the current model. In addition, differences between mouse and human skin may be responsible for the differences between the tumors in the model and human seborrheic keratoses.

### Example 2: Treatment of KS.FGFR3-S249C mice with SU5402, an inhibitor of tyrosine kinase activity of FGFR3.

As the expression of mutated FGFR3 induced a skin hyperplasia in the K5-(S249C)FGFR3 transgenic mice, the effect of FGFR inhibitors are tested *in vivo* by comparing, in transgenic mice, the lesions treated with a FGFR inhibitor and the non treated lesions. The inventors have then tested whether the FGFR tyrosine kinase inhibitor SU5402 effect on the inhibition of the cell proliferation in the lesions.

SU5402 is an ATP mimetic known to inhibit FGFR1 tyrosine kinase phosphorylation by binding to the ATP-binding site. The inventors found that SU5402 did indeed inhibit FGF1-induced FGFR3 phosphorylation or FGFR3 S249C autophosphorylation in NIH-3T3 cells. They therefore treated with SU5402 the snout of control mice and of 4 to 5-month-old K5-(S249C)FGFR3 transgenic mice who had developed skin verrucous lesion. Since the lesions were symmetric both side were treated: one side with 100µM inhibitor and the other side with acetone, three times a week for 4 weeks. In transgenic mice the proportion of basal cells undergoing proliferation, assayed by incorporation of the 5-bromo-2-deoxyuridine, was significantly reduced in the snout side treated with SU5402 (See figure 7). Moreover, clearly visible resorption of the skin lesions was macroscopically observed on 3 mice of the tested group (n=8). In non transgenic control mice, SU5402 did not reduce the proliferation rate of basal cells.

### Example 3: Cosmetic composition (gel)

The cosmetic composition comprises at least one tyrosine kinase inhibitor specific for FGFR3 (for example, SU5402, Calbiochem®) in association with the following usual compounds for such a kind of composition :
- dibasic sodium phosphate,
- edetate disodium,
- mineral oil,
- fatty acid or wax,
- propylene glycol,
- PEG,
- perfume,
- purified water.

Said compounds will be used by the man skilled in the art with appropriate proportions.

## Claims

1. Use of epidermis lesions of a transgenic animal excluding human, wherein transgenic animal cells express a mutated FGFR3 gene under the control of a promoter targeting the mutated activating FGFR3 expression at the animal's epidermis, as a model for seborrheic keratoses.

2. Use according to claim 1, wherein FGFR3 mutation is selected in the group comprising R248C, S249C, G372C, S373C, Y375C, K652E, K652M, A393E, X809C, X809G, X809L, X809R and X809W.

3. Use according to claim 1, wherein said promoter is selected in the group comprising Keratin 1, Keratin 5, Keratin 6, Keratin 10, Keratin 14, Involucrin, Loricrin and Transglutaminase 1 promoters.

4. Use according to claim 1, wherein said promoter targets the basal layer of the epidermis.

5. Use according to claim 4, wherein the promoter is selected in the group comprising Keratin 5 and Keratin 14 promoters.

6. Use according to claim 1, wherein said promoter targets the suprabasal layer of the epidermis.

7. Use according to claim 6, wherein the promoter is selected in the group comprising Keratin 1, Keratin 10, Involucrin and Loricrin promoters.

8. Use according to any of the preceding claims, wherein the transgenic animal is a mouse.

9. Use according to any of the preceding claims, wherein the transgenic animal is a mouse expressing the FGFR3 S249C mutated gene under the control of the bovine Keratin 5 or the human Keratin 14 promoter.

10. A method for screening compounds for treating seborrheic keratoses comprises:
- administering a compound to be tested to the transgenic animal as used in claims 1 to 9,
- detecting the inhibition of cell proliferation and/or the inhibition of the activitation of FGFR3 and/or resorption of the epidermis lesions,
- comparing said treated lesions with a control.

11. Use of a tyrosine kinase inhibitor specific for FGFR3 for the manufacture of a medicament for treating seborrheic keratoses.

12. Use of a tyrosine kinase inhibitor specific for FGFR3 in cosmetic compositions.

13. Use according to claim 11 or 12, wherein the tyrosine kinase inhibitor specific for FGFR3 is a chemical compound, a siRNA or an antibody.

14. A cosmetic treatment comprising the application on the seborrheic keratosis of a composition comprising an effective amount of at least a tyrosine kinase inhibitor specific for FGFR3.
